# EUROPÄISCHE PATENTSCHRIFT

(11) **EP 3 265 779 B1**
(45) Veröffentlichungstag und Bekanntmachung des Hinweises auf die Patenterteilung: **09.08.2023**
(21) Anmeldenummer: 16708082.9
(22) Anmeldetag: 02.03.2016
(51) Int. Cl.: G01N 21/21, G01P 13/00, G01N 21/47, G01N 33/50, G01N 21/49

(54) **VERFAHREN ZUR OPTISCHEN DETEKTION EINER BEWEGUNG IN EINER BIOLOGISCHEN PROBE MIT RÄUMLICHER AUSDEHNUNG**
METHOD FOR OPTICAL DETECTION OF A MOVEMENT IN A BIOLOGICAL SAMPLE WITH A SPATIAL EXTENT
PROCÉDÉ DE DÉTECTION OPTIQUE D'UN MOUVEMENT DANS UN ÉCHANTILLON BIOLOGIQUE À EXPANSION SPATIALE

(30) Priorität: 06.03.2015 DE 102015003019
(43) Veröffentlichungstag der Anmeldung: 10.01.2018
(60) Teilanmeldung: 23182756.9
(73) Patentinhaber: Fraunhofer-Gesellschaft zur Förderung der angewandten Forschung e.V., 80686 München (DE); Universität des Saarlandes, 66123 Saarbrücken (DE)
(72) Erfinder: ZIMMERMANN, Heiko, 97295 Waldbrunn (DE); STRACKE, Frank, 66123 Saarbrücken (DE); LE HARZIC, Ronan, 57915 Woustviller (FR)
(74) Vertreter: v. Bezold & Partner Patentanwälte - PartG mbB
(86) Internationale Anmeldenummer: PCT/EP2016/000377
(87) Internationale Veröffentlichungsnummer: WO 2016/142043

(56) Entgegenhaltungen:
- WO-A1-2014/123156
- US-A- 5 061 075
- US-A1- 2006 105 357
- POMARICO J ET AL: "Compact device for assessment of microorganism motility", REVIEW OF SCIENTIFIC INSTRUMENTS, AIP, MELVILLE, NY, US, Bd. 75, Nr. 11, 1. November 2004 (2004-11-01), Seiten 4727-4731, XP012071874, ISSN: 0034-6748, DOI: 10.1063/1.1809266
- J A COLE ET AL: "Laser speckle spectroscopy-a new method for using small swimming organisms as biomonitors", BIOIMAGING, Bd. 4, Nr. 4, 1. Dezember 1996 (1996-12-01), Seiten 243-253, XP55277248, GB ISSN: 0966-9051, DOI: 10.1002/1361-6374(199612)4:4<243::AID-BIO3 >3.3.CO;2-5
- P. P. DE TOMBE ET AL: "Force and velocity of sarcomere shortening in trabeculae from rat heart. Effects of temperature", CIRCULATION RESEARCH., Bd. 66, Nr. 5, 1. Mai 1990 (1990-05-01), Seiten 1239-1254, XP55277139, US ISSN: 0009-7330, DOI: 10.1161/01.RES.66.5.1239
- S M Baylor ET AL: "A LARGE BIREFRINGENCE SIGNAL PRECEDING CONTRACTION IN SINGLE TWITCH FIBRES OF THE FROG", J. Physiol, 1. Januar 1977 (1977-01-01), Seiten 141-162, XP55278022, Gefunden im Internet: URL:http://www.ncbi.nlm.nih.gov/pmc/articl es/PMC1307751/pdf/jphysiol00824-0153.pdf
- NODA NAOKI ET AL: "A new microscope optics for laser dark-field illumination applied to high precision two dimensional measurement of specimen displacement", REVIEW OF SCIENTIFIC INSTRUMENTS, AIP, MELVILLE, NY, US, Bd. 79, Nr. 2, 14. Februar 2008 (2008-02-14), Seiten 23704-23704, XP012115026, ISSN: 0034-6748, DOI: 10.1063/1.2839914
- KONG FANTING ET AL: "Focusing of light through scattering media", PHOTONS PLUS ULTRASOUND: IMAGING AND SENSING 2011, SPIE, 1000 20TH ST. BELLINGHAM WA 98225-6705 USA, vol. 7899, no. 1, 10 February 2011 (2011-02-10), pages 1-6, XP060007440, DOI: 10.1117/12.873836 [retrieved on 2011-02-17]
- LODAHL P ET AL: "Transport of quantum noise through random media", ARXIV.ORG, CORNELL UNIVERSITY LIBRARY, 201 OLIN LIBRARY CORNELL UNIVERSITY ITHACA, NY 14853, 14 October 2004 (2004-10-14), XP080171782, DOI: 10.1103/PHYSREVLETT.94.153905
- TUCHIN V V: "TISSUE OPTICS: TOMOGRAPHY AND TOPOGRAPHY", PROCEEDINGS OF SPIE, IEEE, US, vol. 3726, 6 October 1998 (1998-10-06), pages 168-198, XP008003055, DOI: 10.1117/12.341389 ISBN: 978-1-62841-730-2

## Beschreibung

Die Erfindung betrifft ein Verfahren zur optischen In-Vitro-Detektion einer Bewegung in einer biologischen Probe mit räumlicher Ausdehnung.

Aus der Praxis ist bekannt, dass ein Bedarf an einer Überwachung der aktiven Dynamik von geschlossenen und dreidimensionalen Zell- und Gewebekulturen in Bereichen wie der Entwicklungsbiologie, der Toxizitätstests und der pharmazeutischen Forschung besteht.

Beispielsweise werden aus embryonalen Stammzellen gezüchtete Gewebestücke, die sich zu einem Muskelgewebe differenziert haben, im Rahmen von Toxizitätstests genutzt, um die Schädlichkeit von einer zu prüfenden Substanz zu prüfen. Hierbei wird untersucht, ob eine auf das Muskelgewebe aufgebrachte Substanz die Muskelkontraktionen des Muskelgewebes beeinflusst, was ein Indikator für die Toxizität der Substanz sein kann. Hierfür sind Messverfahren erforderlich, um eine Bewegung, z. B. eine Kontraktion, in einer solchen dreidimensionalen biologischen Probe in Form eines Zellhaufens zu detektieren. Typische Durchmesser solcher Zellhaufen betragen 100 bis 400 µm, wobei auch Durchmesser im Millimeterbereich möglich sind.

Diese Studien werden derzeit in erster Linie durch visuelle Beobachtungen und selten durch Videomikroskopie mit anschließender Bildanalyse durchgeführt. Erstere sind zeitaufwändig und immer mit subjektiver Einschätzung verbunden. Letztere hat den Nachteil, dass komplexe abbildende Optiken und eine komplexe Bildanalyse verbunden mit einem erheblichen Rechenaufwand erforderlich sind. Nachteilig ist ferner ihre inhärente Empfindlichkeit gegenüber geringfügigen Verschiebungen.

Nichtoptische Methoden wie Impedanzmessungen funktionieren nur im Kontakt zur Probe. Wenn die Probeform jedoch von der Ebene (adhärente Monolage) abweicht oder gar dreidimensional ist, noch dazu frei in einem Medium schwimmt, sind die vorgenannten Techniken nicht anwendbar.

Automatisierte bildgebende Verfahren haben den Nachteil, dass z. B. bei einer Schärfentiefe von 10 µm und der vorstehend genannten typischen Größe der Zellhaufen von 100 bis 400 µm 10 bis 40 Bildebenen der Probe durchgemessen werden müssten. Bei einer typischen Mindestmessdauer von ca. 10 Sekunden, um eine Bewegung detektieren zu können, und der zusätzlich für die Neupositionierung bzw. Fokussierung benötigten Zeit sind derartige Verfahren nicht geeignet, schnell eine Vielzahl von Proben zu überwachen.

Eine serielle Messung von großen Probenzahlen ist auf Grund der durch die Zeitskalen der biologischen Dynamik recht langen Beobachtungsdauer generell nicht angezeigt. In der Praxis besteht jedoch der Bedarf, eine derartige Bewegungsdetektion an einer Vielzahl voneinander getrennter, z. B. in einer Multiwell-Platte, auch als Mikrotiterplatte bezeichnet, z. B. mit in 96 oder 384 Kavitäten (engl. Wells) gelagerten Proben, durchzuführen. Bildgebende Verfahren sind für eine parallele Messung einer Vielzahl solcher Proben nicht geeignet, da es aus geometrischen und bauraumtechnischen Gründen schwierig zu realisieren ist, an jeder Kavität der Multiwell-Platte eine bildgebende optische Vorrichtung anzuordnen.

Das Dokument WO 2014/123156 A1 offenbart die Detektion der Bewegung in einer Probe aus Muskelzellen durch die Berechnung des Kontrastes eines abgebildeten Speckle-Musters.

Der Artikel: J A COLE ET AL: "Laser speckle spectroscopy-a new method for using small swimming organisms as biomonitors", BIOIMAGING, Bd. 4, Nr. 4, Dezember 1996, Seiten 243-253 offenbart eine punktuelle Messung eines Speckle-Musters mit anschließender Fourier-Transformation (FFT) zur Analyse der Bewegung von Mikro-Organismen.

Es ist somit eine Aufgabe der Erfindung, ein verbessertes Verfahren zur Detektion einer Bewegung in einer biologischen Probe mit räumlicher Ausdehnung bereitzustellen, mit dem Nachteile herkömmlicher Techniken vermieden werden können. Der Erfindung liegt insbesondere die Aufgabe zugrunde, ein robustes, kontaktfreies Verfahren zur Bewegungsdetektion bereitzustellen, das keine komplexe Bildanalyse erfordert. Es ist eine weitere Aufgabe der Erfindung, ein Verfahren bereitzustellen, das sich für die parallele Analyse einer Vielzahl von Proben in einer Screening-Umgebung eignet.

Diese Aufgaben werden durch ein Verfahren mit den Merkmalen des unabhängigen Anspruchs 1 gelöst. Vorteilhafte Ausführungsformen und Anwendungen der Erfindung ergeben sich aus den abhängigen Ansprüchen und werden in der folgenden Beschreibung unter teilweiser Bezugnahme auf die Figuren näher erläutert.

Die Erfindung beruht auf der technischen Erkenntnis, dass für eine kontaktlose Überwachung optische Methoden am geeignetsten sind und dass, da der genaue Ort einer möglichen Bewegung in der Probe nicht von vornherein bekannt ist, es erforderlich ist, die gesamte Probe zu beleuchten. Da die Probe in der Regel eine erhebliche Ausdehnung in der Tiefe, d. h. größer als die Schärfentiefe einer Abbildungsoptik, besitzt, müssen die Wechselwirkungen des Lichts mit der Probe auf seiner gesamten Strecke durch die Probe kumuliert gemessen werden. Transmissionsmethoden sind daher ungeeignet, da hier gerade der nicht-wechselwirkende Teil des Lichts gemessen würde und die zu erwartenden Schwankungen von einem hohen Hintergrund und Rauschen begleitet wären, der die Technik unempfindlich machen würde. Der beschriebene Ansatz beruht daher auf der Erfassung der Streu-, Polarisations- und/oder erfindungsgemäß der Beugungsstrahlung der belichteten Probe, wobei nach den durch die Probenbewegung hervorgerufenen Schwankungen in dem Teil des Lichts gesucht wird, der durch Wechselwirkung mit der Probe in ihrer Strahlrichtung, ihrem Polarisationszustand und/oder erfindungsgemäß ihrem Beugungsmuster verändert wurde. Dazu ist es vorteilhaft, das transmittierende Licht durch geeignete Filter von der Streu-, Polarisations- und/oder Beugungsstrahlung abzutrennen. Im Rahmen der vorliegenden Erfindung wird statt des Begriffs "Strahlung" auch der Begriff "Licht" verwendet. Beide Begriffe sind im Rahmen der vorliegenden Erfindung als gleichbedeutend anzusehen und umfassen elektromagnetische Strahlung im sichtbaren, IR- und UV-Bereich.

Gemäß allgemeinen Gesichtspunkten der Erfindung wird somit ein Verfahren zur optischen In-Vitro-Detektion einer Bewegung in einer biologischen Probe mit räumlicher Ausdehnung bereitgestellt.

Das erfindungsgemäße Verfahren umfasst das Bereitstellen einer Aufnahme für die Probe, einer Lichtstrahlenquelle, einer Optik und eines Detektors. Hierbei ist die Optik ausgebildet, die ganze Probe in der Aufnahme mit von der Lichtstrahlenquelle ausgehender Strahlung zu beleuchten und zumindest einen Teil der Strahlung der Lichtstrahlenquelle, die an einer beliebigen Stelle innerhalb der Probe durch eine Wechselwirkung mit der Probe in ihrem Beugungsmuster verändert wird, auf eine Detektionsfläche des Detektors zu leiten, so dass die Wechselwirkungen der von der Lichtstrahlenquelle ausgesandten Strahlung mit der Probe auf seiner gesamten Strecke durch die Probe kumuliert gemessen werden. Der Detektor ist ausgebildet, in Abhängigkeit von der detektierten Strahlung ein Messsignal zu erzeugen, dessen zeitlicher Verlauf einen zeitlichen Verlauf der Intensität der detektierten Strahlung angibt und/oder aus dem der zeitliche Verlauf der Intensität der detektierten Strahlung ableitbar ist.

Das erfindungsgemäße Verfahren umfasst ferner das Beleuchten der Probe mit Strahlung der Lichtstrahlenquelle und das Detektieren einer Bewegung in der biologischen Probe in Abhängigkeit von einer zeitlichen Veränderung des Messsignals.

Ein besonderer Vorzug des erfindungsgemäßen Ansatzes ist, dass aus der Messgröße des Verfahrens direkt die Probendynamik ableitbar ist, da die von einer Probenbewegung hervorgerufenen Schwankungen in dem Teil des Lichts, der durch Wechselwirkung mit der Probe im Beugungsmuster verändert wurde, direkt als Schwankungen in dem Messsignal sichtbar sind. Somit kann auf eine aufwändige Verarbeitung der Messdaten, wie dies bei bildgebenden Verfahren der Fall ist, verzichtet werden.

Außerhalb der Erfindung ist es möglich, eine Bewegung in der Probe zu detektieren, falls eine Veränderung des Messsignals einen vorbestimmten Schwellenwert übersteigt. Erfindungsgemäß wird bei der Überprüfung von Kontraktionen in einem Muskelgewebe eine Bewegung detektiert, falls die zeitliche Veränderung des Messsignals eine Periodizität aufweist. Das Verfahren kann unter Verwendung vergleichsweise einfacher optischer Elemente durchgeführt werden. Eine Optik zur Fokussierung auf einzelne Bildebenen und zum sukzessiven Abtasten des Probenvolumens ist nicht notwendig. Daher kann die Vorrichtung zur Durchführung des Verfahrens kostengünstig und baulich kompakt ausgeführt sein.

Aufgrund der einfachen Auswertung des Messsignals und des baulich kompakten Aufbaus ist das Verfahren auch für die parallele Überwachung einer Vielzahl von Proben geeignet und lässt sich prozesseffizient in Screening-Umgebungen bzw. in automatisierte Hochdurchsatzverfahren, z. B. High-Throughput Screening-Verfahren, integrieren.

Gemäß einer besonders bevorzugten Ausführungsvariante ist der Detektor einkanalig ausgeführt bzw. gibt der Detektor ein einkanaliges Messsignal aus. Das Messsignal gibt vorzugsweise nur die pro Zeiteinheit auf die Detektorfläche eintreffende Strahlungsintensität an. Eine Bewegung in der Probe kann somit direkt anhand einer zeitlichen Veränderung bzw. Schwankung des Signals detektiert werden.

Der Detektor ist erfindungsgemäß ein nicht bildgebender Detektor oder ein Detektor mit nicht ortsaufgelöstem Messsignal, z. B. ein nicht ortsauflösender Photodetektor, beispielsweise eine Photodiode. Derartige Detektoren sind kompakt und kostengünstig.

Unter einer biologischen Probe mit räumlicher Ausdehnung wird eine dreidimensionale biologische Probe, z. B. in Form einer dreidimensionalen Zell- und/oder Gewebekultur bzw. eines Zellhaufens, verstanden.

Der Durchmesser der biologischen Probe kann mindestens 50 Mikrometer (µm) in mindestens einer Raumrichtung, weiter vorzugsweise mindestens 50 Mikrometer in allen Raumrichtungen betragen und beträgt oft mehr als 100 µm in allen Raumrichtungen.

Der Durchmesser der Probe liegt vorzugsweise im Bereich von 100 µm bis 5 mm, weiter vorzugsweise im Bereich von 100 µm bis 1 mm. Die biologische Probe ist eine Probe aus lebenden Zellen, d. h. aus Zellen, die eine Aktivdynamik aufweisen, d. h. Zellen, die eine Bewegung auslösen können.

Unter der Detektion einer Bewegung in der biologischen Probe soll insbesondere eine Bewegung innerhalb der Probe oder eine Bewegung eines Probenbestandteils der biologischen Probe verstanden werden. Mit anderen Worten sollen allgemein dynamische Phänomene in bzw. innerhalb biologischer Proben mit räumlicher Ausdehnung detektiert werden können. Bei Zellkulturen aus Muskelzellen können derartige Bewegungen beispielsweise durch Kontraktion einzelner Muskelzellen ausgelöst werden.

Bei der biologischen Probe kann es sich nicht erfindungsgemäß um eine Probe aus freischwimmenden Mikroorganismen, beispielsweise Spermien, handeln. In diesem Fall kann das Verfahren zur Detektion der Bewegung der freischwimmenden Mikroorganismen genutzt werden, beispielsweise im Falle von Spermien zur Bestimmung der Spermienmotilität.

Die Optik kann eine auf der Beleuchtungsseite angeordnete Beleuchtungsoptik umfassen, mittels der die Strahlung der Lichtstrahlenquelle auf die ganze Probe geleitet wird, um die Probe vollständig und möglichst gleichmäßig zu beleuchten. Die Optik kann ferner eine Detektionsoptik umfassen, mittels der das von der Probe ausgesandte Licht, das durch eine Wechselwirkung mit der Probe in ihrer Strahlrichtung, ihrem Polarisationszustand und/oder ihrem Beugungsmuster verändert wird, auf eine Detektionsfläche des Detektors geleitet wird. Diese funktionale Eigenschaft der Optik kann dabei unter Verwendung eines oder mehrerer zweckmäßig angeordneter und ausgestalteter bekannter optischer Bauelemente und Komponenten, wie z. B. Filter, Linsen, Blenden, refraktiver Elemente etc., realisiert werden, was nachfolgend anhand weiterer Ausführungsbeispiele erläutert wird.

Eine vorteilhafte Ausführungsvariante sieht hierbei vor, dass die Optik ausgebildet ist und/oder der Detektor relativ zum Beleuchtungsstrahlengang und der Probe so angeordnet ist, dass keine Strahlengänge existieren, bei denen durch die Probe transmittierte Strahlung der Lichtstrahlenquelle auf den Detektor trifft und/oder bei denen Licht der Lichtstrahlenquelle unter Umgehung der Probe auf den Detektor trifft.

Gemäß dieser Ausführungsvariante trifft somit nur dasjenige Licht der Lichtstrahlenquelle auf den Detektor, das durch eine Wechselwirkung mit der Probe in seiner Strahlrichtung, seinem Polarisationszustand und/oder Beugungsmuster verändert wurde, während die Optik verhindert, dass Transmissionsstrahlung oder die Probe umgehende Strahlung die Detektionsfläche des Detektors trifft. Dadurch werden störende Hintergrundsignale reduziert und die Empfindlichkeit der Messung erhöht.

Erfindungsgemäß wird eine Bewegung in der Probe anhand einer Veränderung des Beugungsmusters erkannt. Gemäß dieser Variante erzeugt die Lichtstrahlenquelle kohärentes Licht. Ferner ist die Optik ausgebildet, z. B. mittels eines Raumfilters, einen Randbereich eines Beugungsmusters, das von durch die Probe gebeugtem Licht der Lichtstrahlenquelle erzeugt wird, auf den Detektor abzubilden. Eine Bewegung innerhalb der Probe erzeugt eine Änderung des Beugungsmusters, erfindungsgemäß eines Speckle-Musters. Untersuchungen im Rahmen der Erfindung haben gezeigt, dass die Änderung des Beugungsmusters in seinem Zentrum schwierig zu messen ist, da die relative Änderung der Strahlungsintensität klein ist. Im Randbereich ist die Änderung jedoch zuverlässig zu erkennen und kann z. B. zu einer kurzzeitigen Änderung von einem lokalen Beugungsmaximum zu einem lokalen Beugungsminimum oder vice versa im Beugungsmuster führen. Bei einem Beugungsmuster enthält jeder Punkt des Musters die Beugungsinformation der gesamten Probe.

Erfindungsgemäß umfasst die Optik eine Lochblende, die so zwischen der Probe und dem Detektor angeordnet ist, dass ein Loch der Lochblende an dem Randbereich des von der Probe erzeugten Beugungsmusters angeordnet ist.

Unter einer Lochblende wird eine lochförmige Öffnung, vorzugsweise eine kleine lochförmige Öffnung verstanden und vorzugsweise ohne Linse. Lochblenden dienen dem örtlich begrenzten Aufsammeln von Licht. Zum gleichen Zweck werden insbesondere in konfokalen Mikroskopen seit langer Zeit die Stirnseiten von optischen Fasern genutzt.

Als Randbereich des Beugungsmusters sollen vorzugsweise alle Beobachtungswinkel gelten, in den kein transmittiertes Licht empfangen wird. Das Beugungsmuster wird durch positive und negative Interferenz von Lichtwellen, die an Objekten, hier der Probe, gebeugt wurden, hervorgerufen. Ob positiv oder negativ interferiert wird, hängt von Objektgröße, Wellenlänge des Lichts und Beobachtungswinkel ab. Licht, das die Probe ohne Wechselwirkung durchläuft, d. h. transmittiertes Licht, ballistische Photonen, hat den Beobachtungswinkel 0° und trifft in der Mitte des Beugungsmusters auf. Das Beugungsmuster wird durch das transmittierte Licht überstrahlt und das S/B (S/B: signal-to-background, Signal-Hintergrund)-Verhältnis und das S/N (S/N: signal-to-noise, Signal-Rausch)-Verhältnis fallen drastisch. Als Randbereich des Beugungsmusters sollen daher vorzugsweise alle Beobachtungswinkel gelten, in den kein transmittiertes Licht empfangen wird. Da hier die ganze Probe bestrahlt wird und die Beleuchtung nicht kollimiert erfolgt, ist der "Empfangsbereich" der transmittierten Strahlung größer als nur ein Punkt.

Gemäß einer weiteren Variante dieser Ausgestaltungsform kann die Lochblende auch mehrere Löcher aufweisen, die relativ zum Beugungsmuster so angeordnet sind, dass sie sich in einem Randbereich des Beugungsmusters befinden. Hierbei sind die Löcher so anzuordnen, dass die Überlagerung der Beugungsstrahlung, die durch die Löcher auf den Detektor trifft, den Beugungseffekt verstärkt und nicht verschlechtert.

Gemäß einer weiteren Variante der Ausgestaltungsform, die eine Bewegung in der Probe anhand einer Veränderung des Beugungsmusters erkennt, kann die Optik eine zwischen der Lichtstrahlenquelle und der Probe angeordnete Blende umfassen, die so ausgeführt ist, dass eine durch eine Blendenöffnung der Blende austretende Strahlung der Lichtstrahlenquelle nicht direkt, d. h. unter Umgehung der Probe, auf das Loch der Lochblende trifft. Ferner kann die Optik ein zwischen der Lichtstrahlenquelle und der Probe angeordnetes refraktives optisches Element, d. h. ein die Strahlung brechendes Element, z. B. eine konvexe Linse oder ein Prisma, aufweisen, das so ausgeführt ist, dass durch das refraktive Element abgelenkte Strahlung nicht direkt, d. h. unter Umgehung der Probe, auf das Loch der Lochblende trifft. Diese Varianten stellen ein kostengünstiges und einfach zu justierendes Beispiel für eine Optik dar, die nur die gebeugte Strahlung zum Loch der Lochblende leitet.

Gemäß einer nicht erfindungsgemäßen Ausgestaltungsform wird eine Bewegung in der Probe anhand einer durch die Bewegung verursachten Schwankung von polarisiertem Licht erkannt. Gemäß dieser Ausgestaltungsform umfasst die Optik einen ersten Polarisationsfilter und einen zweiten Polarisationsfilter, die unterschiedliche Polarisationsrichtungen aufweisen, wobei der erste Polarisationsfilter zwischen Lichtstrahlenquelle und Probe und der zweite Polarisationsfilter zwischen Probe und Detektor angeordnet ist. Eine Bewegung in der Probe führt zu einer geänderten Wechselwirkung des polarisierten Lichts mit der Probe und zu einer Änderung der Polarisationszustände, was zu einer Schwankung im Detektorsignal führt.

Hierbei können der Detektor und der zweite Polarisationsfilter in Bezug auf die Lichtstrahlenquelle auf der gegenüberliegenden Seite der Probe, seitlich von der Probe oder auf der gleichen Seite wie die Lichtstrahlenquelle angeordnet sein.

Gemäß einer weiteren nicht erfindungsgemäßen Ausgestaltungsform wird eine Bewegung in der Probe anhand einer durch die Bewegung verursachten Schwankung des an der Probe gestreuten Lichts erkannt. Hierbei kann der der Detektor zur Epidetektion von Streulicht der Probe auf der gleichen Seite der Probe (1) wie die Lichtstrahlenquelle angeordnet sein. Alternativ kann der der Detektor zur Detektion seitlicher Streustrahlung der Probe in Bezug auf die Richtung des Beleuchtungsstrahlengangs schräg und/oder seitlich von der Probe angeordnet sein.

Diese nicht erfindungsgemäßen Varianten bieten den Vorteil, dass keine optischen Komponenten wie z. B. Blenden notwendig sind, die verhindern, dass Transmissionsstrahlung auf den Detektor trifft.

Ferner kann der Detektor zur Durchlichtdetektion von Streulicht der Probe in Durchlichtrichtung zur Probe angeordnet sein. Gemäß dieser Variante umfasst die Optik eine zwischen der Lichtstrahlenquelle und der Probe angeordnete Blende, die ausgebildet ist, im Beleuchtungsstrahlengang Lichtstrahlen, die als durch die Probe transmittierte Strahlen auf den Detektor treffen würden, zu blockieren. Alternativ oder zusätzlich kann die Optik ein zwischen der Lichtstrahlenquelle und der Probe angeordnetes refraktives optisches Element umfassen, das ausgebildet ist, eine Richtung des Beleuchtungsstrahlengangs so zu verändern, dass durch die Probe transmittierte Lichtstrahlen nicht auf den Detektor treffen.

Ferner ist es vorteilhaft, wenn die Optik einen vor dem Detektor angeordneten Bandpassfilter aufweist, der zur Raumlichtunterdrückung ausgebildet ist. Dadurch kann die Empfindlichkeit der Detektion weiter erhöht werden.

Gemäß einer weiteren vorteilhaften Variante umfasst die Optik, insbesondere die Beleuchtungsoptik, ein Axicon. Die Verwendung eines Axicons bietet den Vorzug einer homogeneren Ausleuchtung räumlich "tiefer" Proben, verglichen mit konvexen Linsen, da sich der Fokus eines Axicons entlang der optischen Achse erstreckt - statt nur in einem Punkt. Ein weiterer Vorzug eines Axicons ist die einfache räumliche Filterung des eingestrahlten (nicht beeinflussten) Lichts, da dieses mit einem konstanten Winkel zur optischen Achse gebrochen wird.

Eine Möglichkeit der erfindungsgemäßen Realisierung sieht vor, dass sich die Probe auf einer Trägermatrix befinden kann. Die Aufnahme der Probe kann somit eine Trägermatrix, vorzugsweise ein Biopolymer, umfassen. Die Probe kann sich ferner in einem hängendem Tropfen befinden. Die Aufnahme der Probe kann somit einen hängenden Tropfen umfasst. Ferner kann die Aufnahme der Probe als eine sich in einem hängenden Tropfen befindliche Trägermatrix, vorzugsweise ein Biopolymer wie z. B. Alginat, ausgeführt sein.

Für die Anwendung des Verfahrens in Screening-Umgebungen kann die Aufnahme für die Probe eine Kavität einer Multiwell-Platte (Mikrotiterplatte) sein. Weiterhin kann die Aufnahme eine Kavität einer Multiwell-Platte sein, die zur Ausbildung eines hängenden Tropfens an den einzelnen Kavitäten ausgebildet ist (sog. Hängender-Tropfen-Multiwell-Platte). Solche Hängender-Tropfen-Multiwell-Platten werden beispielsweise von der Firma InspheroAG, CH-8952 Schlieren unter der Bezeichnung "GravityPLUS^{™} 3D Culture and Assay Platform" angeboten. Auch die Patentschrift EP 2342317 B1 offenbart eine solche Platte.

Vorstehend wurde bereits erwähnt, dass sich das Verfahren besonders für die parallele Überwachung einer Vielzahl von Proben eignet, z. B. von Proben, die im Rahmen von automatisierten Hochdurchsatzverfahren untersucht werden sollen.

Eine vorteilhafte Weiterbildung des Verfahrens sieht daher vor, dass mit diesem eine parallele optische Detektion einer Bewegung in mehreren voneinander getrennten biologischen Proben durchgeführt wird. Hierbei ist die Aufnahme für die biologischen Proben vorzugsweise eine Multiwell-Platte, die eine Mehrzahl von in Reihen und Spalten angeordneten Kavitäten zur Aufnahme der Proben aufweist. Der Detektor ist als Detektorarray, vorzugsweise als Photodiodenarray, ausgeführt, wobei ein Rasterabstand der einzelnen Detektoren einem Rasterabstand der Kavitäten der Multiwell-Platte entspricht.

Die Lichtstrahlenquelle ist ausgeführt, die einzelnen Kavitäten zu beleuchten. Gemäß einer vorteilhaften Variante ist die Lichtstrahlenquelle zur Beleuchtung der Proben in den Kavitäten als ein Laserdiodenarray ausgeführt, wobei ein Rasterabstand der einzelnen Laserdioden dem Rasterabstand der Kavitäten der Multiwell-Platte entspricht. Ein Laserdiodenarray stellt eine platzsparende und energieeffiziente Beleuchtungsquelle dar.

Um einen guten Wärmeabtransport zu ermöglichen, kann die Halterung des Laserdiodenarrays aus einem wärmeleitfähigen Material, vorzugsweise aus Aluminium, ausgeführt sein. Weiterhin kann die Optik gemäß dieser Variante ein Linsenarray, z. B. ein Mikrolinsenarray, umfassen, wobei jede Linse des Linsenarrays einer der Laserdioden zugeordnet ist und die Linsen das Licht der Laserdioden in die Kavitäten leiten.

Anstatt eines Laserdiodenarrays als Lichtstrahlenquelle kann die Lichtstrahlenquelle als herkömmliche Lichtquelle (Laser, Bogenlampe etc.) ausgeführt sein, wobei das Licht der Lichtstrahlenquelle zur Beleuchtung der Proben über ein Lichtfaserbündel in die einzelnen Kavitäten, die die Proben enthalten, eingekoppelt wird. Jede Lichtfaser ist dabei einer Kavität zugeordnet. Dies bietet den Vorteil, dass die Lichtquelle ausreichend beabstandet zur Probe betrieben werden kann, um und eine zu starke Wärmeentwicklung in Probennähe zu vermeiden.

Ferner besteht gemäß einer weiteren alternativen Variante die Möglichkeit, die Multiwell-Platte mit einer Lichtstrahlenquelle flächig zu beleuchten, was eine einfache Umsetzungsvariante darstellt, jedoch Energieeffizienznachteile hat, da die Lichtstrahlenquelle entsprechend leistungsfähig sein muss. Vorteilhaft ist es hierbei, wenn das Licht über eine zweckmässig ausgeführte Kondensoroptik und ggf. winkelabhängige Durchlassfilter parallelisiert und gezielt auf die Kavitäten geleitet wird.

Gemäß einem weiteren nicht erfindungsgemäßen Gesichtspunkt der Erfindung wird eine Vorrichtung zur kontaktfreien In-Vitro-Detektion einer Bewegung in einer biologischen Probe mit räumlicher Ausdehnung bereitgestellt. Die Vorrichtung umfasst eine Aufnahme für die biologische Probe, eine Lichtstrahlenquelle, einen Detektor und eine Optik, die ausgebildet ist, die ganze Probe in der Aufnahme mit von der Lichtstrahlenquelle ausgehenden Strahlung zu beleuchten und zumindest einen Teil der Strahlung der Lichtstrahlenquelle, die an einer beliebigen Stelle innerhalb der Probe durch eine Wechselwirkung mit der Probe in ihrer Strahlrichtung, ihrem Polarisationszustand und/oder ihrem Beugungsmuster verändert wurde, auf eine Detektionsfläche des Detektors zu leiten. Der Detektor ist ausgebildet, in Abhängigkeit von der detektierten Strahlung ein Messsignal zu erzeugen, das den zeitlichen Verlauf der Intensität der detektierten Strahlung angibt und/oder aus dem der zeitliche Verlauf der Intensität der detektierten Strahlung ableitbar ist.

Die Vorrichtung kann ferner eine Auswerteeinheit aufweisen, die eingerichtet ist, eine Bewegung in der biologischen Probe durch Anzeige und/oder Auswertung einer zeitlichen Veränderung der detektierten Strahlung zu detektieren.

Weitere Einzelheiten und Vorteile der Erfindung werden im Folgenden unter Bezug auf die beigefügten Zeichnungen beschrieben. Es zeigen:
- Figur 1: eine stark schematisierte Darstellung eines nicht erfindungsgemäßen Verfahrens und einer nicht erfindungsgemäßen Vorrichtung gemäß einer Ausführungsform;
- Figuren 2A-2E: nicht erfindungsgemäße Ausführungsformen, die Streulicht der Probe zur Bewegungsdetektion verwenden;
- Figuren 3A-3C: nicht erfindungsgemäße Ausführungsformen, die polarisiertes Licht zur Bewegungsdetektion verwenden;
- Figuren 4A-4C: Ausführungsformen der Erfindung, die ein Beugungsmuster der Probe zur Bewegungsdetektion verwenden;
- Figur 5A: eine Probe in Form eines Herzmuskelgewebemodells auf einer Trägermatrix;
- Figur 5B: Darstellung eines Speckle-Musters an zwei aufeinanderfolgenden Zeitpunkten; und
- Figur 5C: einen zeitlichen Verlauf eines beispielhaften Messsignals.

Gleiche Teile sind in den Figuren mit denselben Bezugszeichen versehen und werden nicht gesondert beschrieben.

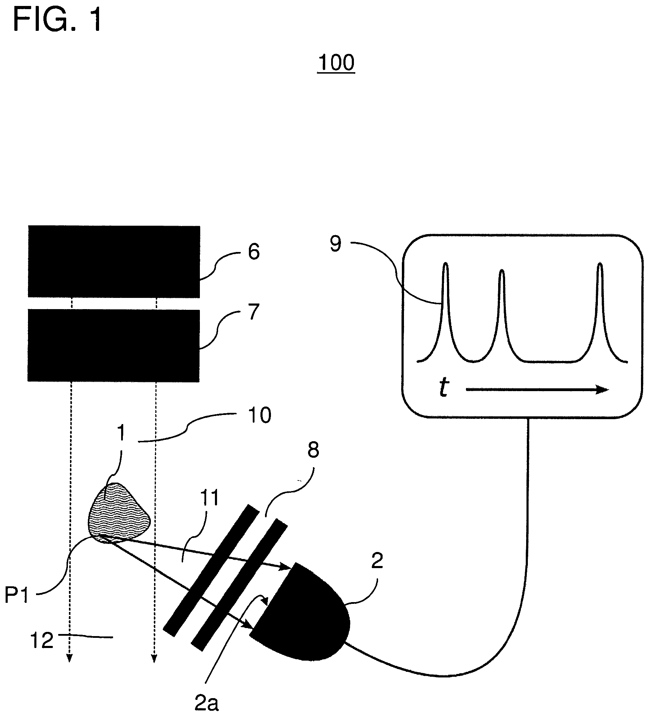

Figur 1 zeigt eine stark schematisierte Darstellung eines nicht erfindungsgemäßen Verfahrens und einer nicht erfindungsgemäßen Vorrichtung.

Zur Durchführung des Verfahrens wird eine Vorrichtung 100 zur optischen In-Vitro-Detektion einer Bewegung in einer biologischen Probe 1 mit räumlicher Ausdehnung bereitgestellt.

Die Vorrichtung 100 umfasst eine Aufnahme (nicht dargestellt) für die dreidimensionale Probe 1, eine Lichtstrahlenquelle 6, eine Optik 7, 8 und einen Detektor 2.

Die Aufnahme ist nicht auf eine bestimmte Art von Aufnahmen beschränkt, sondern kann je nach Anwendungszweck und Art der Probe zweckmäßig, z. B. als Träger, Trägerplatte, als Gefäß, als Kavität einer Multiwellplatte, oder als Trägermatrix in Form eines Biopolymers, z. B. Alginat, auf dem die Probe gezüchtet wird, ausgeführt sein.

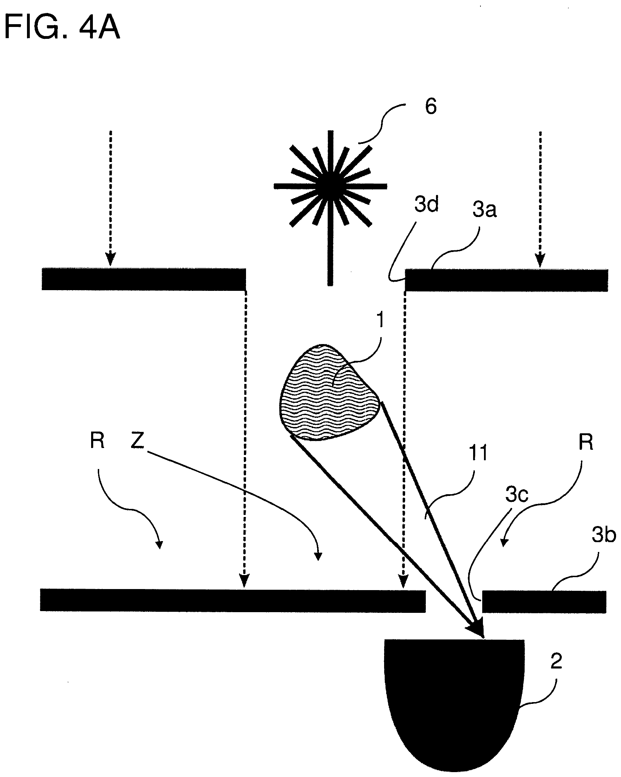

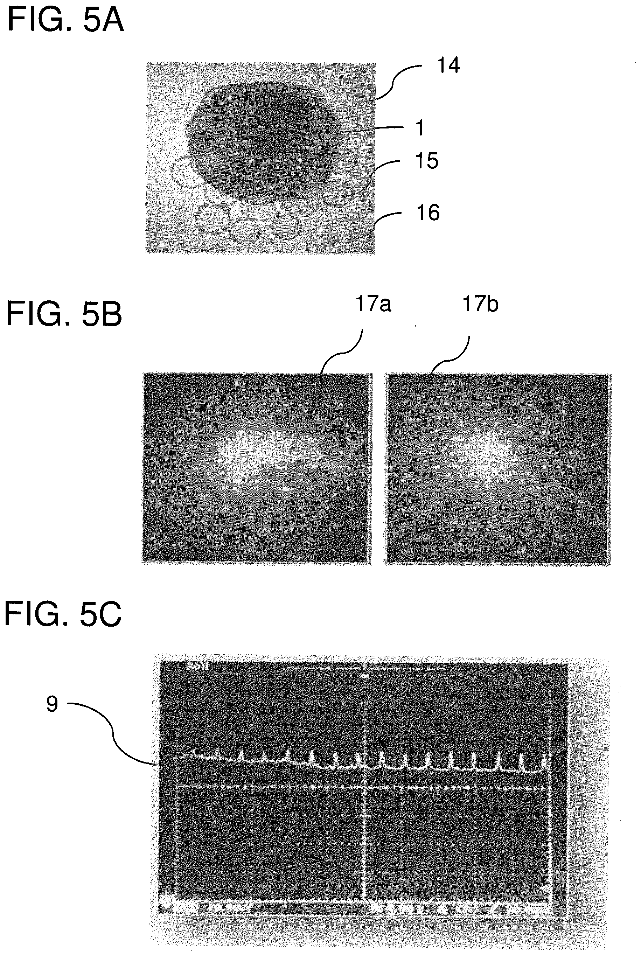

Die Lichtquelle 6 kann, muss aber keine kohärente Lichtquelle, z. B. ein Laser, sein. Lediglich die Ausführungsvarianten, die ein Beugungsmuster der Probe zur Bewegungsdetektion (vgl. Figuren 4A bis 4C, 5A bis 5C) verwenden, benötigen kohärente Strahlung.

Die Darstellung der Optik 7, 8 in Figur 1 ist lediglich schematisch und soll die funktionale Eigenschaft der Optik illustrieren und keine bestimmten optischen Elemente darstellen, da prinzipiell eine Vielzahl konkreter optischer Ausführungsvarianten möglich ist, von denen einige beispielhaft in den nachfolgenden Figuren beschrieben sind.

Diese funktionale Eigenschaft der Optik kann dabei unter Verwendung eines oder mehrerer zweckmäßig angeordneter und ausgestalteter bekannter optischer Bauelemente und Komponenten, wie z. B. Filter, Linsen, Blenden, refraktiver Elemente etc., realisiert werden.

Die Optik 7, 8 umfasst eine auf der Beleuchtungsseite angeordnete Beleuchtungsoptik 7, mittels der die Strahlung 10 der Lichtstrahlenquelle 6 auf die ganze Probe 1 geleitet wird, um die Probe vollständig und möglichst gleichmäßig zu beleuchten. Die Beleuchtungsoptik kann hierfür geeignete optische Elemente bzw. Komponenten zur Strahlformung, wie Blenden, Linsen und/oder Filter, umfassen. Vorteilhaft ist insbesondere die Verwendung eines Axicons.

Die Optik 7, 8 umfasst ferner eine Detektionsoptik 8, mittels der das von der Probe ausgesandte Licht 11, d. h. Licht der Lichtstrahlenquelle 6, das durch eine Wechselwirkung mit der Probe 1 in seiner Strahlrichtung, seinem Polarisationszustand und/oder Beugungsmuster verändert wurde, auf eine Detektionsfläche 2a des Detektors 2 geleitet wird. In Figur 1 ist beispielsweise ein Streuvorgang im Punkt P1 dargestellt, bei dem Licht 11 in Richtung des Detektors 2 gestreut wird und mittels der Detektionsoptik 8 auf den Detektor 2 abgebildet wird. Da die ganze Probe 1 gleichmäßig beleuchtet wird, kann die Wechselwirkung des einfallenden Lichts 10 an jedem beliebigen Punkt innerhalb der Probe 1 stattfinden, so dass die Wechselwirkungen des Lichts mit der Probe auf seiner gesamten Strecke durch die Probe kumuliert vom Detektor 2 gemessen werden.

Die Detektionsoptik 8 kann ferner optische Elemente enthalten, die sicherstellen, dass Licht, das nicht durch eine Wechselwirkung mit der Probe in seiner Strahlrichtung, seinem Polarisationszustand und/oder Beugungsmuster verändert wurde, nicht auf den Detektor 2 trifft. Beispielsweise kann die Detektionsoptik 8 einen Bandpassfilter aufweisen, der vor dem Detektoreingang angeordnet ist und das Raumlicht herausfiltert oder unterdrückt, jedoch Licht mit einer Wellenlänge der Lichtstrahlenquelle 6 durchlässt. Dies setzt voraus, dass eine monochromatische Lichtstrahlenquelle verwendet wird oder ein entsprechender Filter am Ausgang der Lichtstrahlenquelle angeordnet ist, um die Probe nur mit einer bestimmten Lichtwellenlänge zu beleuchten.

Ferner kann die Detektionsoptik 8 mittels Blenden, Linsen etc. Strahlengänge blockieren, bei denen durch die Probe 1 transmittierte Strahlung der Lichtstrahlenquelle 6 auf den Detektor treffen würde und/oder bei denen Licht der Lichtstrahlenquelle 6 unter Umgehung der Probe auf den Detektor 2 treffen würde.

Zusätzlich oder alternativ kann der Detektor auch so angeordnet sein, dass kein Durchlicht 12 auf seine Detektorfläche 2a trifft, z. B. durch seitliche Anordnung des Detektors 2 relativ zum Beleuchtungsstrahlengang 10, wie in Figur 1 illustriert.

Der Detektor 2 ist ausgebildet, in Abhängigkeit von der detektierten Strahlung 11 ein Messsignal 9 zu erzeugen, dessen zeitlicher Verlauf einen zeitlichen Verlauf einer Intensität der detektierten Strahlung 11 angibt.

Das Detektorsignal 9 entspricht somit einer Volumenmessung (engl. full-volume measurement) der Probe. Der Detektor 2 ist erfindungsgemäß ein nicht bildgebender Detektor oder ein Detektor mit nicht ortsaufgelöstem Messsignal, vorzugsweise einkanalig, so dass nur eine Messgröße 9 erzeugt wird, die der Schwankung der vom Detektor pro Zeiteinheit erfassten Lichtintensität entspricht. Der Detektor 2 ist z. B. eine herkömmliche Photodiode 2.

Mit der in Figur 1 illustrierten Vorrichtung 100 wird die Probe 1 beleuchtet und das entsprechende Messsignal 9 ausgewertet.

Findet nun eine Bewegung in der Probe 1 statt, z. B. eine Kontraktion im Falle von gezüchtetem Muskelgewebe, dann ändert sich durch die Bewegung in der Probe auch die Wechselwirkung der Probe 1 mit dem auf die Probe treffenden Licht, d. h., der Anteil des auf die Probe treffenden Lichts 11, das durch eine Wechselwirkung mit der Probe 1 in seiner Strahlrichtung, seinem Polarisationszustand und/oder erfindungsgemäß seinem Beugungsmuster verändert wird, ändert sich und erzeugt eine Änderung im Detektorsignal 9. Gemäß dem Verfahren kann somit eine Bewegung in der biologischen Probe anhand der Schwankung des Detektorsignals 9 detektiert werden.

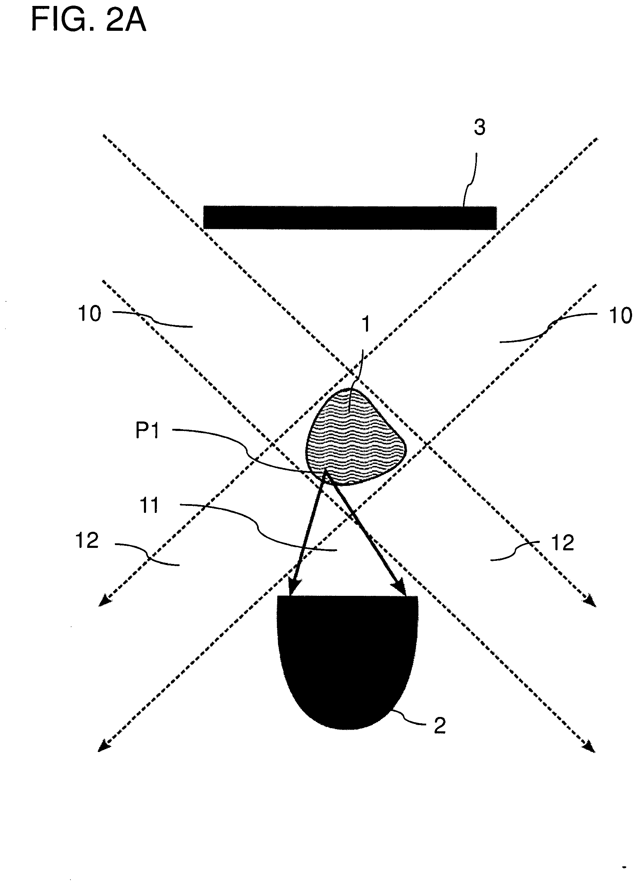

Nachfolgend werden einige beispielhafte Ausführungsformen der Erfindung beschrieben, die konkrete Ausgestaltungen des in Figur 1 dargestellten Lösungsansatzes darstellen. In den Figuren 2A bis 3C ist die Lichtstrahlenquelle 6 nicht dargestellt, befindet sich aber oberhalb der gezeigten Optik- und Detektoranordnung, was aus dem Strahlengang 10 ersichtlich ist.

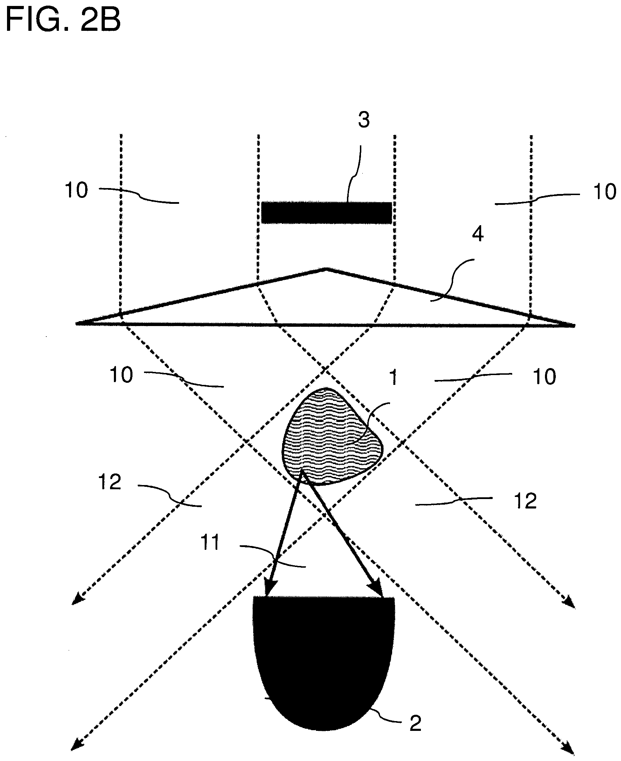

Die Figuren 2A bis 2E zeigen nicht erfindungsgemäße Ausführungsformen, die Streulicht der Probe zur Bewegungsdetektion verwenden. In den Figuren 2A und 2B ist der Detektor 2 zur Durchlichtdetektion von Streulicht 11 der Probe 2 in Durchlichtrichtung zur Probe 1 angeordnet. Um zu verhindern, dass durch die Probe 2 transmittierte Strahlen auf den Detektor 2 treffen, ist in Figur 2A eine Blende 3 angeordnet, die Lichtstrahlen blockiert, die als durch die Probe 2 transmittierte Strahlen auf den Detektor 2 treffen würden oder die seitlich an der Probe vorbei auf den Detektor 2 treffen könnten. Die Beleuchtungsoptik in Form der Blende 3 lässt also nur solche durch die Probe transmittierte Strahlung 12 zu, die nicht auf den Detektor 12 trifft.

Die Besonderheit der in Figur 2B dargestellten Ausführungsvariante ist, dass statt einer großen Blende 3 eine kleinere Blende 3 verwendet wird, der ein refraktives optisches Element, z. B. eine Linse, ein Axicon etc. nachgeordnet ist, das den durch die Blende 3 durchgelassen Beleuchtungsstrahlengang 10 so in seiner Richtung verändert, dass durch die Probe 2 transmittierte Strahlen 12 nicht auf den Detektor 2 treffen können.

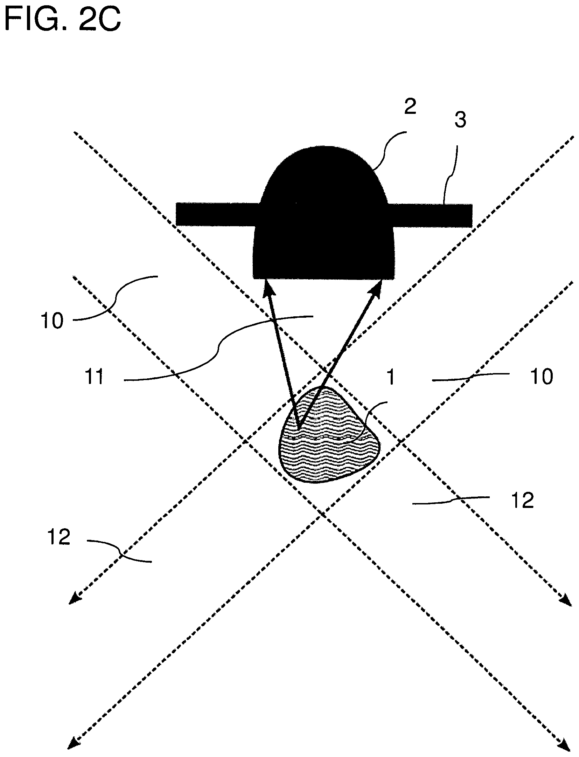

Die Besonderheit der in Figur 2C dargestellten Ausführungsvariante ist, dass der Detektor 2 zur Epidetektion von Streulicht in Form von Rückstrahlung der Probe 1 auf der gleichen Seite der Probe 1 wie die Lichtstrahlenquelle 6 angeordnet ist, wobei die Detektorfläche 2a wiederum der Probe 2 zugewandt ist. Dies bietet den Vorteil, dass keine Blende zum Blockieren von Durchlicht notwendig ist. Vorliegend ist dennoch eine den Detektor 2 umgebende Blende vorgesehen, die Einflüsse von störenden Lichteinflüssen reduzieren kann.

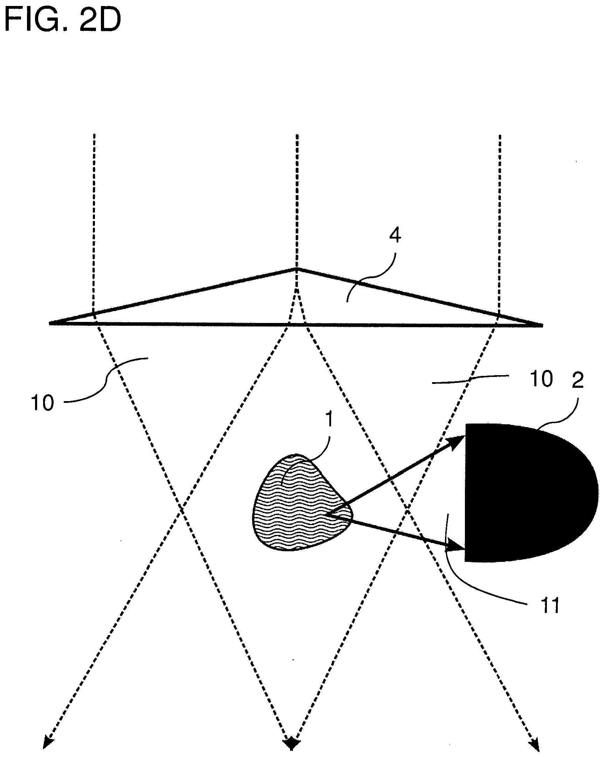

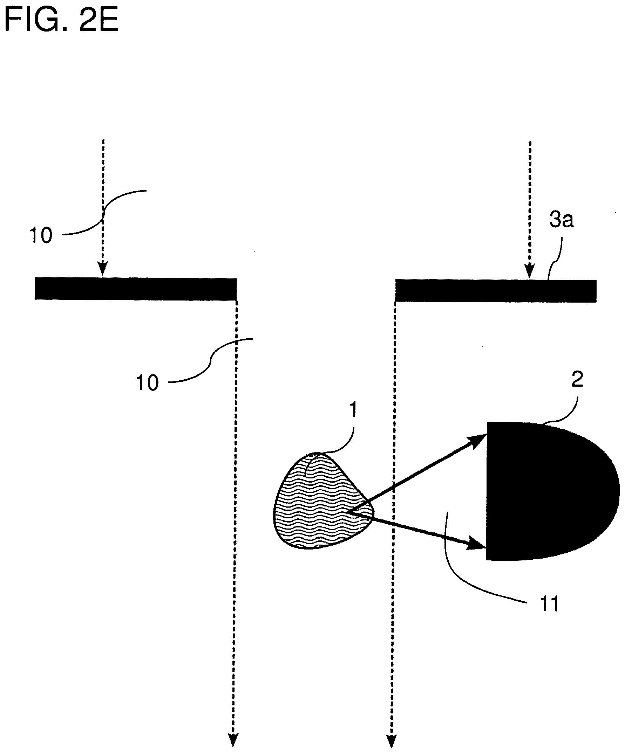

Die Besonderheit der in den Figuren 2D und 2E dargestellten Ausführungsvarianten ist, dass seitliches Streulicht 11 detektiert wird. Hierzu ist der Detektor 2 seitlich zur Beleuchtungsrichtung angeordnet. In Figur 2D ist zwischen Lichtstrahlenquelle und Probe 1 ein refraktives optisches Element, z. B. eine Linse oder Prisma 4, angeordnet, das die Richtung des Strahlengangs so verändert, dass Licht des Beleuchtungsstrahlengangs 10 nicht direkt, d. h. unter Umgehung der Probe 1, auf den Detektor 2 treffen kann.

Die in Figur 2E dargestellte Ausführungsvariante unterscheidet sich von der Variante in Figur 2E dadurch, dass statt des refraktiven optischen Elements 4 eine Blende 3a verwendet wird, die eine Öffnung zur Verengung des Strahlengangs 10 aufweist, so dass wiederum Licht des Beleuchtungsstrahlengangs 10 nicht direkt, d. h. unter Umgehung der Probe 1, auf den Detektor 2 treffen kann.

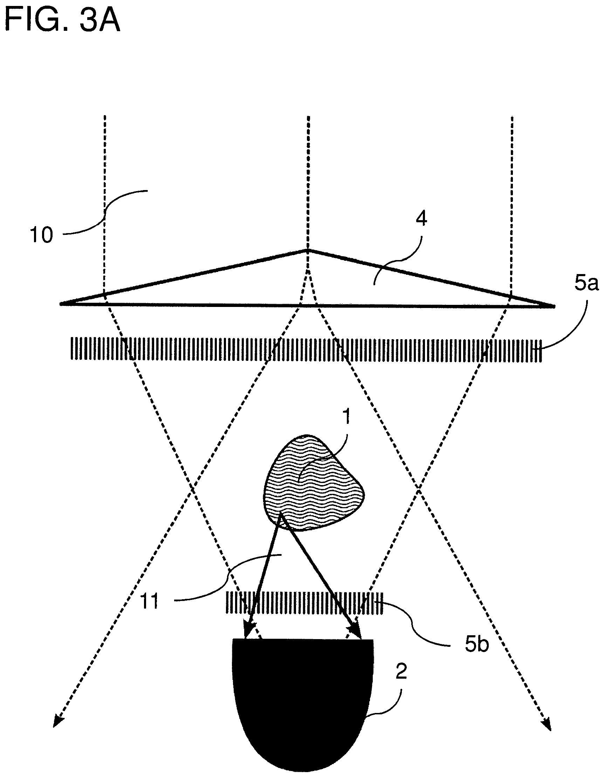

Die Figuren 3A bis 3C zeigen nicht erfindungsgemäße Ausführungsformen, die polarisiertes Licht zur Bewegungsdetektion verwenden. Die Beleuchtungsoptik umfasst hierzu einen ersten Polarisationsfilter 5a, der zwischen der Lichtstrahlenquelle und der Probe 1 angeordnet ist. Die Detektionsoptik umfasst einen zweiten Polarisationsfilter 5b, der eine unterschiedliche Polarisationsrichtung im Vergleich zum ersten Polarisationsfilter 5a aufweist und zwischen Probe 1 und Detektor 2, vorzugsweise am Detektoreingang, angeordnet ist.

Der Detektor 2 und der zweite Polarisationsfilter 5b können in Bezug auf die Lichtstrahlenquelle auf der gegenüberliegenden Seite der Probe 1, seitlich von der Probe 1 oder auf der gleichen Seite wie die Lichtstrahlenquelle angeordnet sein, was durch die unterschiedlichen Varianten in den Figuren 3A bis 3C dargestellt ist.

Aufgrund der unterschiedlichen Polarisationsrichtung der beiden Polarisationsfilter 5a und 5b lässt der Polarisationsfilter 5b nur Licht durch, das durch eine Wechselwirkung mit der Probe "depolarisiert" wurde. Die Anordnung der beiden Polarisationsfilter 5a und 5b stellt somit sicher, dass kein durch die Probe transmittiertes Licht oder Licht, dass die Probe umgangen hat, detektiert wird.

Durch eine Bewegung in der Probe ändert sich der Anteil an depolarisiertem Licht und führt zu einer Schwankung im Detektorsignal, so dass aus der Schwankung des Detektorsignals wiederum direkt eine Bewegung in der Probe 1 erkannt werden kann.

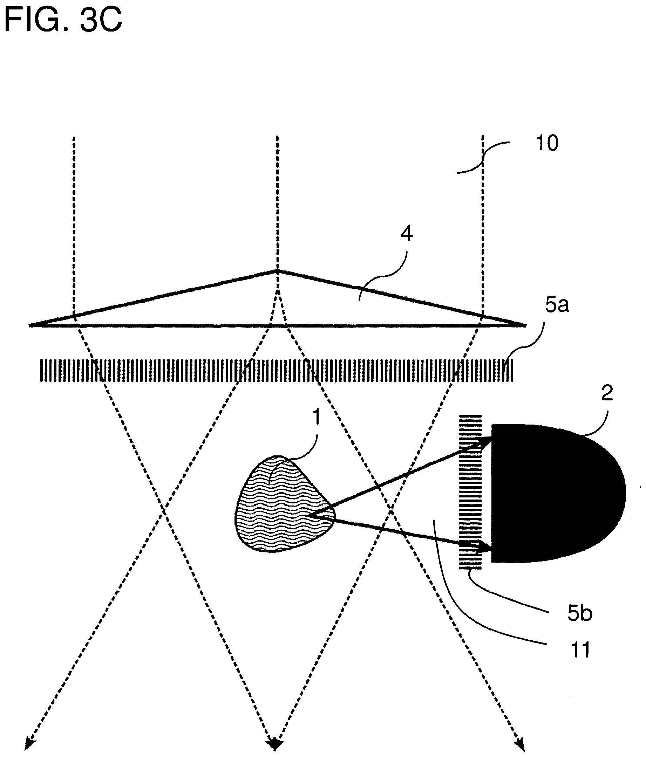

Zur Reduzierung von Streulichteffekten kann vor dem ersten Polarisationsfilter 5a ein refraktives optisches Element 4, z. B. eine konvexe Linse oder ein Prisma, angeordnet sein, das den Beleuchtungsstrahlengang 10 auf die Probe fokussiert (Figuren 3A und 3C).

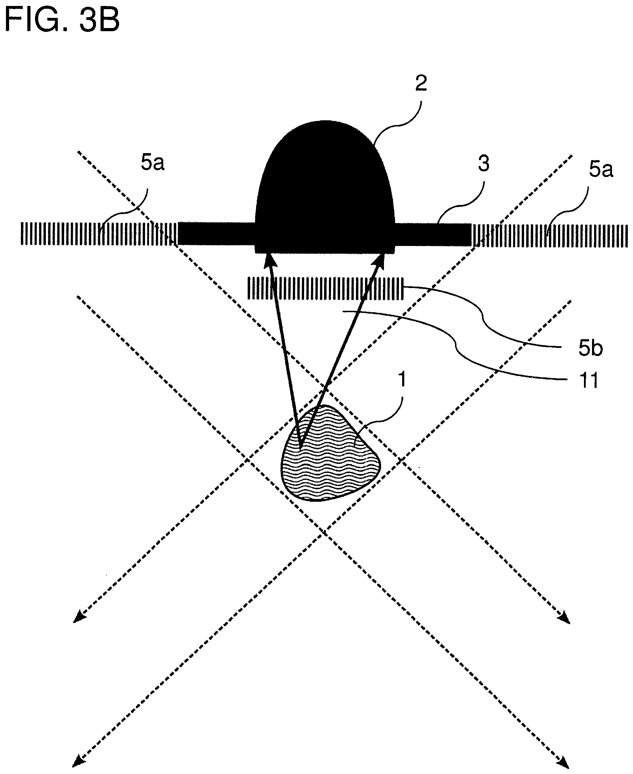

In der Ausführungsvariante der Figur 3B ist der Detektor 2 zur Epidetektion von Streulicht in Form von Rückstrahlung der Probe 1 auf der gleichen Seite der Probe 1 wie die Lichtstrahlenquelle 6 angeordnet. Hier kann eine den Detektor 2 umgebende Blende 3 vorgesehen sein, die Einflüsse von störenden Lichteinflüssen reduzieren kann.

Die Figuren 4A bis 4C zeigen Ausführungsformen der Erfindung, die ein Beugungsmuster der Probe in Form eines Speckle-Musters zur Bewegungsdetektion verwenden. Die Lichtquelle 6 ist eine kohärente Lichtquelle, z. B. eine Laserdiode. Das an der Probe gebeugte Licht der Lichtstrahlenquelle 6 erzeugt ein Beugungsmuster mit einem Zentrum Z hoher Intensität und einem Randbereich R niedriger Intensität.

Die Detektionsoptik umfasst eine vor dem Detektor 2 angeordnete Lochblende 3b, die so zwischen der Probe 1 und dem Detektor 2 angeordnet ist, dass ein Loch 3c der Lochblende 3b in dem Randbereich R des von der Probe erzeugten Beugungsmusters angeordnet ist.

Gemäß der Variante der Figur 4A umfasst die Beleuchtungsoptik eine zwischen der Lichtstrahlenquelle und der Probe angeordnete Blende 3a, die so ausgeführt ist, dass eine durch eine Blendenöffnung 3d der Blende 3a austretende Strahlung der Lichtstrahlenquelle 6 nicht direkt auf das Loch 3c der Lochblende 3b trifft.

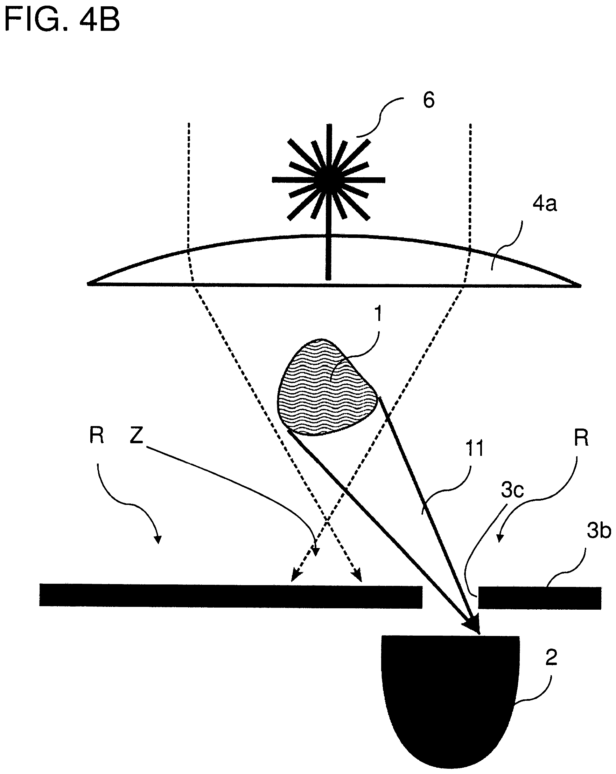

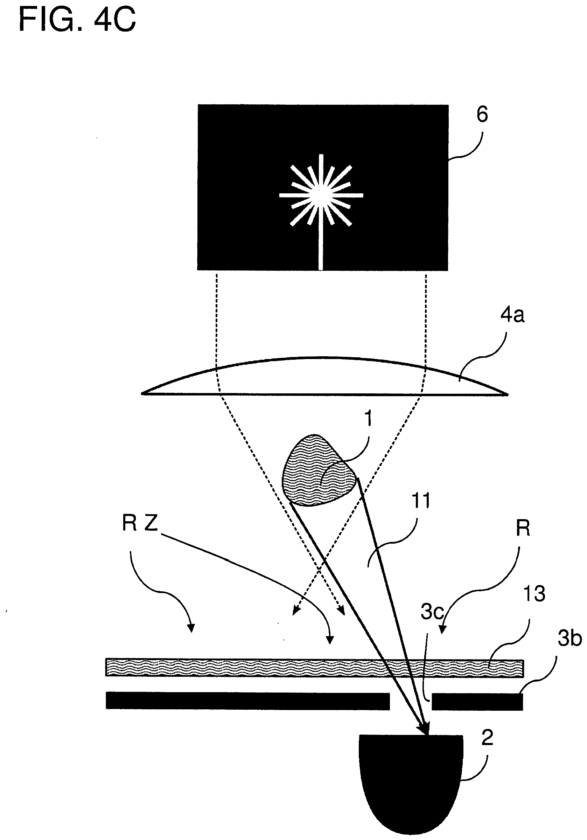

Gemäß den Varianten der Figuren 4B und 4C umfasst die Beleuchtungsoptik ein zwischen der Lichtstrahlenquelle 6 und der Probe 1 angeordnetes refraktives optisches Element 4a, z. B. eine konvexe Linse, das so ausgeführt ist, dass durch das refraktive optische Element 4a gebeugte Strahlung nicht direkt, d.h. unter Umgehung der Probe, auf das Loch 3c der Lochblende 3b trifft.

In der Variante der Figur 4C ist ferner ein Bandpassfilter 13 zwischen Probe 1 und Lochblende 3b angeordnet, der Raumlicht, das nicht der Wellenlänge der Lichtstrahlenquelle 6 entspricht, herausfiltert. Mit dem in Figur 4C gezeigten Aufbau lassen sich derzeit Herzmuskelschläge mit einer der visuellen Beobachtung (am Mikroskop) vergleichbaren Empfindlichkeit nachweisen.

Figur 5A zeigt beispielhaft eine Probe 1 in Form eines Zellhaufens. Der Zellhaufen befindet sich in einem hängenden Tropfen, von dem in Figur 5A nur ein Teil zu sehen ist, der in einer Kavität einer Hängender-Tropfen-Multititer-Platte ausgebildet ist. Die Probe ist besteht aus einem aus Stammzellen ausdifferenzierten Herzmuskelgewebemodell, das auf Trägerbeads 15 in Form von Alginat anhaftet. Die in Figur 5A gezeigte Probe hat einen Durchmesser von ca. 1 Millimeter.

Die Probe 1 wird mit einer Laserdiode mit Licht der Wellenlänge 650nm vollständig beleuchtet. Die auf unterschiedlichen Größenskalen strukturierte Probe 1 beugt das kohärente Licht auf vielfältige Weise und erzeugt in Durchlichtrichtung ein komplexes Beugungsmuster (sog. "Speckle-Muster"). Die durch die lokalen Herzmuskelkontraktionen hervorgerufenen geringfügigen Verformungen dieser Strukturen im Gewebe führen zu einer Veränderung des gesamten Speckle-Musters.

Figur 5B zeigt beispielhaft die Bilder 17a und 17b, die zwei unterschiedliche Zustände eines Speckle-Musters bei minimaler und maximaler Auslenkung der Kontraktion zeigen. Die Bilder 17a und 17b dienen lediglich zur Verdeutlichung und stammen von einem anderen Experiment und zeigen nicht das Speckle-Muster der Probe 1 aus Figur 5A. Das Messprinzip ist jedoch das gleiche. Über einen Raumfilter, z. B. die Lochblende 3b, wird eine Stelle im Randbereich R des Beugungsmusters 17a, 17b auf den Detektor 2 abgebildet. Die Veränderung des Musters 17a, 17b führt nun zu einer schwankenden Lichtmenge, die von der Lochblende 3b durchgelassen wird, und somit zu einem schwankenden Detektorsignal 9, was in Figur 5C dargestellt ist. Die periodische Schwankung des Signals 9 entspricht den periodischen Kontraktionen im Muskelgewebe. Die Darstellung in Figur 5C dient lediglich der Verdeutlichung, zeigt aber wiederum kein Messsignal, das bei der Beleuchtung der in Figur 1A gezeigten Probe gemessen wurde.

Obwohl die Erfindung unter Bezugnahme auf bestimmte Ausführungsbeispiele beschrieben worden ist, ist es für einen Fachmann ersichtlich, dass verschiedene Änderungen ausgeführt werden können und Äquivalente als Ersatz verwendet werden können, ohne den Bereich der Erfindung zu verlassen. Zusätzlich können viele Modifikationen ausgeführt werden, ohne den zugehörigen Bereich zu verlassen. Folglich soll die Erfindung nicht auf die offenbarten Ausführungsbeispiele begrenzt sein, sondern soll alle Ausführungsbeispiele umfassen, die in den Bereich der beigefügten Patentansprüche fallen. Insbesondere beansprucht die Erfindung auch Schutz für den Gegenstand der Unteransprüche unabhängig von den in Bezug genommenen Ansprüchen.

## Patentansprüche

1. Verfahren zur optischen In-Vitro-Detektion einer Bewegung in einer biologischen Probe (1) mit räumlicher Ausdehnung in Form einer dreidimensionalen Zell- und/oder Gewebekultur oder eines Zellhaufens, wobei die Probe (1) lebende Muskelzellen aufweist, umfassend die Schritte:
a) Bereitstellen einer Aufnahme für die Probe (1), einer Lichtstrahlenquelle (6), einer Optik (7, 8) und eines Detektors (2),
a1) wobei die Lichtstrahlenquelle (6) kohärentes Licht erzeugt;
a2) wobei die Optik (7, 8) ausgebildet ist, die ganze Probe (1) in der Aufnahme mit von der Lichtstrahlenquelle (6) ausgehender Strahlung zu beleuchten und zumindest einen Teil der Strahlung (11) der Lichtstrahlenquelle (6), die an einer beliebigen Stelle innerhalb der Probe (1) durch eine Wechselwirkung mit der Probe (1) in ihrem Beugungsmuster verändert wird, auf eine Detektionsfläche (2a) des Detektors (2) zu leiten, und
a3) wobei der Detektor (2) ausgebildet ist, in Abhängigkeit von der detektierten Strahlung ein Messsignal (9) zu erzeugen, dessen zeitlicher Verlauf einen zeitlichen Verlauf einer Intensität der detektierten Strahlung (11) angibt und/oder aus dem der zeitliche Verlauf der Intensität der detektierten Strahlung (11) ableitbar ist;
a4) wobei der Detektor (2) ein nicht bildgebender Detektor oder ein Detektor mit nicht ortsaufgelöstem Messsignal ist;
a5) wobei die Optik (7, 8) ausgebildet ist, einen Randbereich eines Specklemusters, das von durch die Probe (1) gebeugtem Licht der Lichtstrahlenquelle (6) erzeugt wird, auf den Detektor (2) abzubilden; und
a6) wobei die Optik (7, 8) eine Lochblende (3b) umfasst, die so zwischen der Probe (1) und dem Detektor (2) angeordnet ist, dass ein Loch (3c) der Lochblende (3b) in dem Randbereich (R) des von der Probe erzeugten Specklemusters angeordnet ist
b) Beleuchten der Probe (1) mit Strahlung der Lichtstrahlenquelle (6); und
c) Detektieren einer Bewegung in der biologischen Probe (1) in Abhängigkeit von einer zeitlichen Veränderung des Messsignals (9);
wobei eine Bewegung in der Probe (1) detektiert wird, falls die zeitliche Veränderung des Messsignals (9) eine Periodizität aufweist,
wobei eine periodische Schwankung des Messsignals (9) periodischen Kontraktionen der Muskelzellen entspricht.

2. Verfahren nach Anspruch 1, **dadurch gekennzeichnet, dass** ein Durchmesser der Probe (1)
a) mindestens 50 Mikrometer (pm) in mindestens einer Raumrichtung ist; oder
b) im Bereich von 100 µm bis 1 mm liegt.

3. Verfahren nach einem der vorhergehenden Ansprüche, **dadurch gekennzeichnet,**
a) **dass** der Detektor (2) ein einkanaliges Messsignal (9) ausgibt; und/oder
b) **dass** der Detektor (2) eine Photodiode ist.

4. Verfahren nach einem der vorhergehenden Ansprüche, **dadurch gekennzeichnet, dass** die Optik (7, 8) ausgebildet ist und/oder der Detektor (2) relativ zum Beleuchtungsstrahlengang (10) und der Probe (1) so angeordnet ist, dass keine Strahlengänge existieren, bei denen durch die Probe (1) transmittierte Strahlung (12) der Lichtstrahlenquelle (6) auf den Detektor (2) trifft und/oder bei denen Licht der Lichtstrahlenquelle (6) unter Umgehung der Probe (1) auf den Detektor trifft.

5. Verfahren nach einem der vorhergehenden Ansprüche, **dadurch gekennzeichnet, dass** die Optik (7, 8) umfasst:
a) eine zwischen der Lichtstrahlenquelle (6) und der Probe (1) angeordnete Blende (3a), die so ausgeführt ist, dass eine durch eine Blendenöffnung (3d) der Blende (3a) austretende Strahlung der Lichtstrahlenquelle (6) nicht direkt auf das Loch (3c) der Lochblende (3b) trifft, und/oder
b) ein zwischen der Lichtstrahlenquelle (6) und der Probe (1) angeordnetes refraktives optisches Element (4a), das so ausgeführt ist, dass durch das refraktive Element (4a) gebeugte Strahlung nicht direkt auf das Loch (3c) der Lochblende (3b) trifft.

6. Verfahren nach einem der vorhergehenden Ansprüche, **dadurch gekennzeichnet, dass** die Optik (7, 8) einen vor dem Detektor (2) angeordneten Bandpassfilter (13) aufweist, der zur Raumlichtunterdrückung ausgebildet ist.

7. Verfahren nach einem der vorhergehenden Ansprüche, **dadurch gekennzeichnet, dass** die Aufnahme der Probe eine Trägermatrix, vorzugsweise ein Biopolymer (15), umfasst.

## Claims

1. Method for optical in vitro detection of a movement in a biological sample (1) with a spatial extent in the form of a three-dimensional cell and/or tissue culture or a cell cluster, comprising the steps:
a) providing a receptacle for the sample (1), a light beam source (6), optics (7, 8; 3, 3a, 3b, 4, 5a, 5b) and a detector (2),
a1) wherein the light beam source (6) generates coherent light; and
a2) wherein the optics (7, 8) is configured to illuminate the whole sample (1) in the receptacle with radiation emanating from the light beam source and to guide at least a part of the radiation (11) from the light beam source (6), which is changed in its diffraction pattern at any point within the sample (1) through interaction with the sample (1), onto a detection surface (2a) of the detector (2), and
a3) wherein the detector (2) is configured to generate a measurement signal (9) depending on the detected radiation, the time profile of which specifies a time profile of an intensity of the detected radiation (11) and/or from which the time profile of the intensity of the detected radiation (11) can be derived;
a4) wherein the detector (2) is a non-imaging detector or a detector with a non-spatially resolved measurement signal;
a5) wherein the optics (7, 8) is configured to map an edge area of a speckle pattern generated by light from the light beam source that is diffracted by the sample, on the detector (2);
a6) wherein the optics comprise a pinhole aperture (3b) arranged between the sample (1) and the detector (2) in such a way that a pinhole (3c) of the pinhole aperture (3b) is arranged in the edge area (R) of the speckle pattern generated by the sample;
b) illuminating the sample (1) with radiation from the light beam source; and
c) detecting a movement in the biological sample (1) depending on a temporal change of the measurement signal (9) ;
wherein a movement in the sample (1) is detected if a temporal change of the measurement signal (9) has periodicity,
wherein a periodic variation of the measurement signal corresponds to periodic contractions of the muscle cells.

2. Method according to claim 1, **characterised in that** a diameter of the sample
a) is at least 50 micrometres (pm) in at least one spatial direction, or
b) lies within a range of 100 µm to 1 mm.

3. Method according to one of the preceding claims, **characterised in that**
a) the detector (2) emits a single-channel measurement signal (9); and/or
b) the detector (2) is a photodiode.

4. Method according to one of the preceding claims, **characterised in that** the optics (7, 8) is configured and/or the detector (2) is arranged relative to the illumination beam path (10) and the sample (1) in such a way that no beam paths exist where the radiation (12) of the light beam source (6) transmitted through the sample (1) reaches the detector (2) and/or where light from the light beam source reaches the detector whilst bypassing the sample.

5. Method according to one of the preceding claims , **characterised in that** the optics (7,8) comprises:
a) an aperture (3a) arranged between the light beam source and the sample that is designed in such a way that radiation from the light beam source (6) existing through an aperture opening (3d) of the aperture (3a) does not reach the pinhole (3c) of the pinhole aperture (3b) directly, and/or
b) a refractive optical element (4a), arranged between a light beam source (6) and the sample (1), is designed in such a way that radiation diffracted by the refractive element (4a) does not reach the pinhole (3c) of the pinhole aperture (3b) directly.

6. Method according to one of the preceding claims, **characterised in that** the optics comprises a bandpass filter (13) arranged before the detector (2), which is designed for suppressing room light.

7. Method according to one of the preceding claims, **characterised in that** the receptacle for the sample comprises a carrier matrix, preferably a biopolymer (15).

## Revendications

1. Procédé de détection optique in vitro d'un mouvement dans un échantillon biologique (1) à expansion spatiale sous la forme d'une culture cellulaire et/ou tissulaire tridimensionnelle ou d'un amas de cellules, dans lequel l'échantillon (1) présente des cellules musculaires vivantes, comprenant les étapes :
a) de fourniture d'un logement pour l'échantillon (1), d'une source de rayonnement lumineux (6), d'une optique (7, 8) et d'un détecteur (2),
a1) dans lequel la source de rayonnement lumineux (6) génère une lumière cohérente ;
a2) dans lequel l'optique (7, 8) est réalisée pour éclairer la totalité de l'échantillon (1) dans le logement avec un rayonnement partant de la source de rayonnement lumineux (6) et pour diriger au moins une partie du rayonnement (11) de la source de rayonnement lumineux (6), qui est modifiée, sur un emplacement quelconque à l'intérieur de l'échantillon (1), du fait d'une interaction avec l'échantillon (1) quant à son motif de diffraction, sur une surface de détection (2a) du détecteur (2), et
a3) dans lequel le détecteur (2) est réalisé pour générer, en fonction du rayonnement détecté, un signal de mesure (9), dont l'évolution dans le temps indique une évolution dans le temps d'une intensité du rayonnement (11) détecté et/ou à partir duquel l'évolution dans le temps de l'intensité du rayonnement (11) détecté peut être déduite ;
a4) dans lequel le détecteur (2) n'est pas un détecteur à imagerie ou est un détecteur avec un signal de mesure sans résolution spatiale ;
a5) dans lequel l'optique (7, 8) est réalisée pour reproduire sur le détecteur (2) une zone de bord d'un motif granulaire, qui est généré par une lumière, diffractée par l'échantillon (1), de la source de rayonnement lumineux (6) ; et
a6) dans lequel l'optique (7, 8) comprend un diaphragme à trou (3b) qui est disposé de telle sorte entre l'échantillon (1) et le détecteur (2) qu'un trou (3c) du diaphragme à trou (3b) est disposé dans la zone de bord (R) du motif granulaire généré par l'échantillon
b) d'éclairage de l'échantillon (1) avec un rayonnement de la source de rayonnement lumineux (6) ; et
c) de détection d'un mouvement dans l'échantillon biologique (1) en fonction d'une modification dans le temps du signal de mesure (9) ;
dans lequel un mouvement dans l'échantillon (1) est détecté si la modification dans le temps du signal de mesure (9) présente une périodicité,
dans lequel une fluctuation périodique du signal de mesure (9) correspond à des contractions périodiques des cellules musculaires.

2. Procédé selon la revendication 1, **caractérisé en ce qu'**un diamètre de l'échantillon (1)
a) est d'au moins 50 micromètres (pm) dans au moins une direction spatiale ; ou
b) se situe dans la plage de 100 µm à 1 mm.

3. Procédé selon l'une quelconque des revendications précédentes, **caractérisé en ce**
a) **que** le détecteur (2) émet un signal de mesure monocanal (9) ; et/ou
b) **que** le détecteur (2) est une photodiode.

4. Procédé selon l'une quelconque des revendications précédentes, **caractérisé en ce que** l'optique (7, 8) est réalisée et/ou le détecteur (2) est disposé de telle sorte par rapport au chemin optique d'éclairage (10) et à l'échantillon (1) qu'il n'existe aucun chemin optique, pour lequel un rayonnement (12), transmis par l'échantillon (1), de la source de rayonnement lumineux (6) atteint le détecteur (2) et/ou pour lequel de la lumière de la source de rayonnement lumineux (6) atteint le détecteur en contournant l'échantillon (1).

5. Procédé selon l'une quelconque des revendications précédentes,
**caractérisé en ce que** l'optique (7, 8) comprend :
a) un diaphragme (3a) disposé entre la source de rayonnement lumineux (6) et l'échantillon (1), qui est réalisé de telle sorte qu'un rayonnement, sortant par une ouverture de diaphragme (3d) du diaphragme (3a), de la source de rayonnement lumineux (6) n'atteint pas directement le trou (3c) du diaphragme à trou (3b), et/ou
b) un élément optique réfractif (4a), disposé entre la source de rayonnement lumineux (6) et l'échantillon (1), qui est réalisé de telle sorte qu'un rayonnement diffracté par l'élément réfractif (4a) n'atteint pas directement le trou (3c) du diaphragme à trou (3b).

6. Procédé selon l'une quelconque des revendications précédentes, **caractérisé en ce que** l'optique (7, 8) présente un filtre passe-bande (13) disposé devant le détecteur (2), qui est réalisé pour supprimer la lumière ambiante.

7. Procédé selon l'une quelconque des revendications précédentes, **caractérisé en ce que** le logement de l'échantillon comprend une matrice de support, de préférence un biopolymère (15).
